Europäisches Patentamt

European Patent Office

Office européen des brevets

(11) Veröffentlichungsnummer: **0 369 286**
**A2**

(12) **EUROPÄISCHE PATENTANMELDUNG**

(21) Anmeldenummer: **89120553.6**

(51) Int. Cl.⁵: **A61K 31/55**

(22) Anmeldetag: **07.11.89**

Die Bezeichnung der Erfindung wurde geändert (Richtlinien für die Prüfung im EPA, A-III, 7.3).

(30) Priorität: **17.11.88 DE 3838912**

(43) Veröffentlichungstag der Anmeldung:
**23.05.90 Patentblatt 90/21**

(84) Benannte Vertragsstaaten:
**AT BE CH DE FR GB IT LI LU NL SE**

(71) Anmelder: **Dr. Karl Thomae GmbH**
**Postfach 1755**
**D-7950 Biberach 1(DE)**

(72) Erfinder: **Eberlein, Wolfgang, Dr. Dipl.-Chem.**
**Obere Au 6**
**D-7950 Biberach 1(DE)**
Erfinder: **Engel, Wolfhard, Dr. Dipl.-Chem.**
**Mozartstrasse 13**
**D-7950 Biberach 1(DE)**
Erfinder: **Mihm, Gerhard, Dr. Dipl.-Chem.**
**Nickeleshalde 5/1**
**D-7950 Biberach 1(DE)**
Erfinder: **Rudolf, Klaus, Dr.**
**Marktplatz 38**
**D-7950 Biberach 1(DE)**
Erfinder: **Doods, Henri, Dr.** ·
**Hornsteinweg 7**
**D-7951 Warthausen(DE)**
Erfinder: **Mayer, Norbert, Dr.**
**Friedrich-Ebert-Strasse 66**
**D-7950 Biberach 1(DE)**

(54) **Dibenzazepin-Derivate zur Behandlung von akuten und chronischen obstruktiven Atemwegserkrankungen.**

(57) Die Verbindungen 5,11-Dihydro-11-[[(1-methyl-4-piperidinyl)amino]carbonyl]-6H-dibenz[b,e]azepin-6-on, 5,11-Dihydro-11-[(1-methyl-4-piperidinyl)acetyl]-6H-dibenz[b,e]azepin-6-on und 5,11-Dihydro-11-[(4-methyl-1-piperazinyl)acetyl]-6H-dibenz[b,e]azepin-6-on, ihre Enantiomere und ihre physiologisch verträglichen Säureadditionssalze eignen sich zur Behandlung von akuten und chronischen obstruktiven Atemwegserkrankungen.

EP 0 369 286 A2

## Mittel zur Behandlung von akuten und chronischen obstruktiven Atemwegserkrankungen

Die Erfindung betrifft Mittel zur Behandlung von akuten und chronischen obstruktiven Atemwegserkrankungen, welche die Verbindungen
5,11-Dihydro-11-[[(1-methyl-4-piperidinyl)amino]carbonyl]-6H-dibenz[b,e]azepin-6-on und/oder
5,11-Dihydro-11-[(1-methyl-4-piperidinyl)acetyl]-6H-dibenz[b,e]azepin-6-on und/oder
5,11-Dihydro-11-[(4-methyl-1-piperazinyl)acetyl]-6H-dibenz[b,e]azepin-6-on,
ihre wirksamen Enantiomeren und/oder deren physiologisch verträglichen Salze mit anorganischen oder organischen Säuren enthalten.

In der DE-A-3 402 060.8 (entspricht US-Patent 4 567 178) wurden Verbindungen beschrieben, die als $M_1$-selektive muskarinische Rezeptorantagonisten magensäuresekretionsinhibierende Eigenschaften besitzen und damit vorteilhafterweise zur Behandlung von Magen- und Darmerkrankungen eingesetzt werden können.

Es wurde überraschenderweise gefunden, daß die in den oben angeführten Patenten enthaltenen Verbindungen

A = 5,11-Dihydro-11-[[(1-methyl-4-piperidinyl)amino]carbonyl]-6H-dibenz[b,e]azepin-6-on

B = 5,11-Dihydro-11-[(1-methyl-4-piperidinyl)acetyl]-6H-dibenz[b,e]azepin-6-on,

C = 5,11-Dihydro-11-[(4-methyl-1-piperazinyl)acetyl]-6H-dibenz[b,e]azepin-6-on und ihre physiologisch wirksamen Enantiomeren, z.B. die Verbindung

D = (+)-5,11-Dihydro-11-[[(1-methyl-4-piperidinyl)amino] carbonyl]-6H-dibenz[b,e]azepin-6-on und ihre physiologisch verträglichen Salze mit anorganischen oder organischen Säuren noch gänzlich andersgeartete pharmakologische Eigenschaften besitzen, die ihre Anwendung zur Behandlung von akuten und chronischen obstruktiven Atemwegserkrankungen ermöglichen.

In der Literatur ist ausführlich beschrieben, daß Asthma-Anfälle, so auch chronische Bronchitis und Emphysem, mit nicht-selektiven Antimuskarinika, wie Atropin, behandelt werden können.

Ein großer Nachteil der systemischen Anwendung von nicht-selektiven Antimuskarinika ist die hohe Rate nichttolerierbarer anticholinerger Nebenwirkungen wie Mydriasis, Salivationshemmung, Obstipation und schwerwiegende ZNS-Effekte. Diese Nebenwirkungen schließen den Einsatz nichtselektiver Antimuskarinika in der systemischen Therapie aus.

Im Gegensatz zu Atropin wurden für die oben genannten Substanzen A, B und C und ihre Enantiomere, insbesondere ihre (+)-Enantiomere, überraschenderweise eine ausgeprägte Selektivität für die Hemmung der durch exogen appliziertes Acetylcholin induzierten Bronchokonstriktion gefunden. Der große Abstand erwünschter Effekte an den Bronchien zu den unerwünschten anticholinergen Wirkungen (Herz, Blase) ermöglicht den systemischen Einsatz der Verbindungen A, B und C und ihrer Enantiomere aber auch ihrer physiologisch verträglichen Salze bei verschiedenen Formen von obstruktiven Atemwegserkrankungen, ohne daß mit nicht tolerablen Nebenwirkungen zu rechnen ist.

Die hier beschriebenen broncholytischen Effekte sind auf eine selektive Blockade von muskarinischen Rezeptoren zurückzuführen, die an der glatten Muskulatur der Atemwege lokalisiert sind. Diese muskarinischen Rezeptoren werden auch als $M_{SM}$-Rezeptoren bezeichnet.

Darüberhinaus zeigen die oben genannten Substanzen A, B und C und ihre Enantiomere, insbesondere die Substanz D eine ausgeprägte $M_1$-Selektivität in Rezeptor-Bindungsstudien. $M_1$-Rezeptoren sind in den parasympatischen Ganglien lokalisiert, deren postganglionäre Neurone die glatte Muskulatur der Atemwege versorgen. Eine Blockade dieser $M_1$-Rezeptoren führt zu einer Hemmung der Erregungsübertragung von prä- auf postganglionäre Neuronen, was zu einer Abnahme des vagalbedingten Bronchialtonus oder zu einer Hemmung der Reflex-Bonchokonstriktion führt.

Somit leisten die durch $M_1$-Rezeptor-Blockade vermittelten Effekte der oben genannten Verbindungen zusätzlich einen wesentlichen Beitrag zur Wirksamkeit bei der Behandlung von obstruktiven Atemwegserkrankungen.

Diese Kombination aus Blockade der $M_{SM}$-Rezeptoren der Atemwege und der Blockade der ganglionären $M_1$-Rezeptoren führt zu einer höheren Wirksamkeit und eröffnet damit neue therapeutische Perspektiven in der Behandlung chronischer und akuter Atemwegserkrankungen.

Die folgenden Versuche zeigen die günstigen Eigenschaften der Verbindungen A, B, C und D.


A. Bindungsstudien an muscarinischen Rezeptoren:

Bestimmung des $IC_{50}$-Wertes in vitro

Als Organspender dienten männliche Sprague-Dawley-Ratten mit 180-220 g Körpergewicht. Nach Entnahme von Herz, Submandibularis und Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt (v:v):

| Gesamtherz | 1 : 400 |
| Großhirnrinde | 1 : 500 |
| Submandibularis | 1 : 400 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30°C. Die Inkubationsdauer betrug 45 Minuten. Als Radioligand wurde 0,3 n molar $^3$H-N-Methylscopolamin ($^3$H-NMS, Herz und Submandibularis) oder 1 n molar $^3$H-Pirenzepin ($^3$H-PZ, Großhirn) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS bzw. $H^3$-PZ. Der Anteil der unspezifischen Bindung wurde definiert als jene Radioaktivität, die in Anwesenheit von 1 μ molar Quinuclidinylbenzilat gebunden wurde. Es wurden immer Vierfachbestimmungen vorgenommen. Die $IC_{50}$-Werte der nichtmarkierten Test-substanzen wurden graphisch bestimmt. Sie repräsentieren jene Konzentration der Testsubstanz, bei welcher die spezifische Bindung von $^3$H-NMS bzw. $H^3$-PZ an die muscarinischen Rezeptoren in den verschiedenen Organen um 50 % gehemmt wurde. Die Ergebnisse sind aus der Tabelle I zu ersehen.

B. Hemmung der Acetylcholinwirkung auf Bronchien, Blase und Herzfrequenz.

Am narkotisierten Meerschweinchen wurden 5 Minuten nach Gabe der Prüfsubstanz 10 μg/kg Acetyl-cholin intravenös und gleichzeitig intraarteriell injiziert. Dabei wurde die Herzfrequenz durch extrakorporale Ableitung des EKG, der Ausatemwiderstand nach Konzett-Rössler und die Kontraktion der freigelegten Harnblase direkt registriert. Für die Hemmung der Acetylcholinwirkung an den untersuchten Organen wurden Dosis-Wirkungs-kurven aufgenommen und daraus log $ED_{50}$-Werte bestimmt. Ergebnisse siehe Tabelle II.

Tabelle I

| Rezeptor-Bindungstests, in vitro: | | | |
|---|---|---|---|
| | $IC_{50}$ [n Mol 1$^{-1}$] | | |
| | $^3$H-PZ | $^3$H-NMS | $^3$H-NMS |
| Substanz | Cortex | Herz | Submandibularis |
| | ($M_1$) | ($M_2$) | ($M_3$) |
| Atropin | 1,0 | 3,0 | 2,0 |
| A | 6,0 | 400 | 70 |
| B | 2,5 | 42 | 13 |
| C | 8,0 | 800 | 100 |
| D | 3,0 | 300 | 50 |

Die Ergebnisse der Tabelle I zeigen, daß die Verbindungen A, B, C und D zwischen muscarinischen Rezeptoren verschiedener Gewebe unterscheiden.

Anders als Atropin zeigen die Verbindungen A, B, C und D niedrigere $IC_{50}$-Werte für den Cortex-$M_1$-

3

Rezeptor gegenüber muskarinischen Rezeptoren in Präparaten aus dem Herzen und Submandibularis.

Tabelle II

| Hemmung der Acetylcholinwirkung auf Blase, Bronchien und Herzfrequenz des Meerschweinchens: | | | |
|---|---|---|---|
| | $-\log ED_{50}$ (mol/kg) | | |
| Substanz | Bronchien | Herz | Blase |
| Atropin | 7,96 | 7,70 | 7,89 |
| A | 7,26 | 6,02 | 6,27 |
| B | 6,74 | 6,07 | 6,09 |
| C | 6,56 | 5,75 | 5,27 |
| D | 7,48 | 6,39 | 6,51 |

Aus den pharmakologischen Daten der vorstehenden Tabelle II ergibt sich, daß die Bronchokonstriktion durch die Verbindungen A, B, C und D bereits bei Dosierungen gehemmt wird, bei denen keine Effekte auf Herz und Blase beobachtet werden. Atropin zeigt im Gegensatz dazu keine Selektivität.

Zusammenfassend ist zu sagen, daß die Untersuchungsergebnisse für die Verbindungen A, B, C und D beweisen, daß sie in in-vitro-Rezeptorbindungsstudien eine $M_1$-Selektivität zeigen und daß die Substanzen in funktionellen Untersuchungen die cholinerg induzierte Bonchokonstriktion stärker hemmen als Frequenzeffekte am Herzen oder die Blasenkontraktion. Diese $M_1$ und $M_{SM}$-Bronchoselektivität ermöglicht die systemische Behandlung von Krankheiten, die durch eine Atemwegsobstruktion gekennzeichnet sind, z. B. zur Behandlung aller Formen von Asthma, von chronischer Bronchitis und des Emphysem.

Die Verbindungen A, B, C, D, ihre physiologisch wirksamen Enantiomere und ihre physiologisch verträglichen Salze mit anorganischen oder organischen Säuren können sowohl als Monopräparate als auch in Kombination miteinander zu den oben genannten Zwecken verwendet werden. Die Verbindungen A, B, C, D und ihre Enantiomere werden hierzu in an sich bekannter Weise in die üblichen pharmazeutischen Zubereitungsformen, z. B. in Lösungen, Injektionslösungen, Inhalationslösungen, Suppositorien, Tabletten, Dragées oder Kapseln eingearbeitet.

Dosierungen:

Die Tagesdosis liegt bei intravenöser Verabreichung im allgemeinen zwischen 0,010 bis 0,15 mg/kg, vorzugsweise 0,02 und 0,1 mg/kg, bei oraler Verabreichung zwischen 0,10 bis 1,5 mg/kg, vorzugsweise 0,2 und 1,0 mg/kg Körpergewicht, die gegebenenfalls in Form mehrerer, vorzugsweise 1 bis 3 Einzelgaben, zur Erzielung des gewünschten Ergebnisses verabreicht wird.

Die Verbindungen A, B und C werden, wie in der US-Patentschrift 4 567 178 beschrieben, hergestellt.

Die Verbindungen A, B und C liegen hierbei als Racemate vor.

Es wurde nun gefunden, daß beispielsweise im Falle der Substanz A das Enantiomere mit dem spezifischen optischen Drehwert von $[\alpha]_D^{20} = +290.8$ (c = 0,5; in Methanol) die biologisch wirksame Form darstellt.

Die Auftrennung des Racemats in die optisch aktiven Antipoden läßt sich nach bekannten Verfahren durchführen, beispielsweise unter Verwendung einer optisch aktiven Säure. Als optisch aktive Säuren kommen vor allem die L-(+)- oder D-(-)-Weinsäure, eines ihrer Derivate, wie die (+)- oder (-)-Diacetylweinsäure, (+)- oder (-)-Monomethyltartrat oder auch die (+)-Camphersäure in Betracht.

Bei der Synthese des (+)-Enantiomeren der Verbindung A hat sich die Trennung bereits auf der Stufe der Ausgangsverbindung 5,11-Dihydro-11-[(6H-dibenz[b,e]azepin-6-on]-carbonsäure als günstig erwiesen. Diese (+)-5,11-Dihydro-11-[6H-dibenz[b,e]azepin-6-on]-carbonsäure ergibt dann, nach Umsetzung mit 4-Amino-1-methyl-piperidin, unter Anwendung von Methoden, die ohne Racemisierung ablaufen, das gewünschte (+)-Enantiomere D der Verbindung A.

Die Auftrennung der racemischen 5,11-Dihydro-11-[6H-dibenz[b,e]azepin-6-on]-carbonsäure in ihre beiden Enantiomere erfolgt unter Verwendung einer optisch aktiven Base; als solche kommen vor allem Chinin, Chinidin oder R-(+)- und S-(-)-N,N-Dimethyl-phenyl-ethylamin und R-(+)- und S-(-)-α-Methylbenzylamin in Betracht.

Die racemische 5,11-Dihydro-11-[6H-dibenz[b,e)]azepin-6-on]-carbonsäure wird mit einer der vorstehend genannten optisch aktiven Basen in äquimolaren Mengen in einem Lösungsmittel umgesetzt; die so erhaltenen kristallinen, diastereomeren Salze werden anschließend unter Ausnutzung ihrer verschiedenen Löslichkeiten in bestimmten Lösungsmitteln aufgetrennt. Diese Auftrennung kann in jedem beliebigen Lösungsmittel durchgeführt werden, solange dieses einen ausreichenden Unterschied in der Löslichkeit der Salze aufweist. Es werden vorzugsweise Methanol, Ethanol oder deren Gemische, beispielsweise im Volumenverhältnis 4:1, verwendet.

Aus den so erhaltenen Salzen läßt sich die enantiomere Verbindung D nach den Methoden a) und b) herstellen, ohne daß während der Reaktion eine Racemisierung eintritt:

a) durch Freisetzung der (+)-5,11-Dihydro-11-[6H-dibenz[b,e]azepin-6-on]-carbonsäure und Umsetzung dieser in Gegenwart von N-Methylmorpholin und Chlorameisensäure isobutylester mit 1-Methyl-4-amino-piperidin bei Temperaturen von 0°C bis -60°C oder

b) durch direkte Umsetzung des Salzes, z.B. des Chininsalzes in Gegenwart von 4-Methyl-morpholin und Chlorameisensäureisobutylester, mit 1-Methyl-4-amino-piperidin, gegebenenfalls in einem organischen Lösungsmittel bei Temperaturen zwischen 0°C und -60°C.

Selbstverständlich können auch die Racemate der Verbindungen A, B und C nach der in der US-Patentschrift 4 567 178 beschriebenen Methode in ihre (+)- und (-)-Formen aufgetrennt werden.

Die folgenden Beispiele sollen die Herstellung der oben erwähnten Carbonsäure und die oben angegebenen Verfahrensweisen a) und b) für ihre Weiterverarbeitung verdeutlichen:

## Beispiel 1

### (+)-5,11-Dihydro-11-[6H-dibenz[b,e]azepin-6-on]-carbonsäure

101,3 g (0,4 Mol) (±)-5,11-Dihydro-11-[6H-dibenz[b,e]azepin-6-on]-carbonsäure werden in 1000 ml einer Methanol/Ethanol-Mischung (Volumenverhältnis: 4:1) suspendiert und bei 60°C in eine heiße Lösung von 129,6 g (0,4 Mol) Chinin in 900 ml Methanol/Ethanol (Volumenverhältnis 4:1) eingetropft. Aus dieser Lösung kristallisiert beim Abkühlen ein diastereomeres Chinin-Salz aus, das die (+)-Form in einer 70:30 Anreicherung enthält. Das Chinin-Salz wird nach dem Absaugen analog aus dem gleichen Lösungsmittelgemisch Methanol/Äthanol = 4:1 (v:v) noch weitere sechsmal umkristallisiert. Nach insgesamt siebenmaliger Kristallisation erhält man 24 g eines Chinin-Salzes vom Schmelzpunkt 195°C (Zers.), das die (+)-5,11-Dihydro-11-[6H-dibenz[b,e]azepin-6-on]-carbonsäure in optisch reiner Form enthält.

Zur Gewinnung der freien (+)-Carbonsäure schlämmt man 1,0 g (1,7 mMol) des Chinin-Salzes in 30 ml Wasser auf und säuert mit verdünnter Salzsäure an. Der erhaltene Niederschlag wird abgesaugt, mit Wasser gewaschen, und bei 30°C getrocknet.

Die optisch reine (+)-Carbonsäure schmilzt bei 250-251°C.

## Beispiel 2

Direkte Umsetzung des Chinin-Salzes der (+)-5,11-Dihydro-11-[6H-dibenz[b,e]azepin-6-on]-carbonsäure zur Herstellung von (+)-5,11-Dihydro-11-[[(1-methyl-4-piperidinyl)amino]carbonyl]-6H-dibenz[b,e]azepin-6-on

11,4 g (0,02 Mol) des Chininsalzes der (+)-5,11-Dihydro-11-[6H-dibenz[b,e]azepin-6-on]-carbonsäure werden in 1000 ml Tetrahydrofuran aufgeschlämmt und mit 2,0 g (0,02 Mol) 4-Methyl-morpholin versetzt. Nach dem Abkühlen auf -40°C werden 5,4 g (0,04 Mol) Chlorameisensäureisobutylester zugegeben. Man rührt bei der gleichen Temperatur 30 Minuten nach. Anschließend läßt man in die Reaktionsmischung eine Lö- sung von 5,04 g (0,044 Mol) 4-Amino-1-methyl-piperidin in 20 ml absolutem Tetrahydrofuran eintropfen. Man rührt noch eine Stunde unter Kühlung und läßt weitere 2 Stunden stehen. Die Reaktionsmischung wird am Rotationsverdampfer bei 40°C ein- geengt. Der erhaltene Rückstand wird in Methylenchlorid ge- löst, mit Wasser gewaschen und über Magnesiumsulfat getrock- net. Nach dem Einengen erhält man ein schaumiges Produkt, welches durch Digerieren in Essigester zur Kristallisation gebracht wird.

Man erhält ein farbloses Produkt vom Schmelzpunkt 250-251°C und einem optischen Drehwert von $[\alpha]_D^{20} = +290,8$ (c = 0,5, Methanol).

Ausbeute: 2,0 g (28,6% der Theorie).

Beispiel 3

Umsetzung der (+)-5,11-Dihydro-11-[6H-dibenz[b,e]azepin-6-on]-carbonsäure zur Herstellung von (+)-5,11-Dihydro-11-[[(1-methyl-4-piperidinyl)amino]carbonyl]-6H-dibenz[b,e]azepin-6-on

3,9 g (0,015 Mol) (+)-5,11-Dihydro-11-[6H-dibenz[b,e]azepin-6-on]-carbonsäure werden in 300 ml Tetrahydrofuran aufgeschlämmt und mit 1,5 g (0,015 Mol) N-Methylmorpholin versetzt. Man kühlt auf -40°C ab und läßt in diese Reaktionsmischung 2,04 g (0,015 Mol) Chlorameisensäureisobutylester, gelöst in 40 ml Tetrahydrofuran, eintropfen. Man rührt 30 Minuten bei der gleichen Temperatur und gibt dann langsam eine Lösung von 1,89 g (0,016 Mol) 4-Amino-1-methyl-piperidin in 30 ml Tetrahydrofuran hinzu. Man rührt noch eine Stunde unter Kühlung und läßt dann auf Raumtemperatur kommen. Der Ansatz wird am Rotationsverdampfer bei 40°C eingeengt. Das erhaltene Produkt ist nach dem Umkristallisieren aus Essigester völlig identisch mit dem nach Beispiel 2 erhaltenen Produkt.
Ausbeute: 2,3 g (38,2% der Theorie).
Die optische Reinheit des erhaltenen Endproduktes wurde durch HPLC an chiralem Trägermaterial überprüft.
Es konnte nachgewiesen werden, daß der Gehalt an (+)-Form bei über 99 % liegt und das (-)-Enantiomer unter 1 % vorkommt.
Die folgenden Beispiele veranschaulichen die Herstellung pharmazeutischer Anwendungsformen:

Beispiel I

Tabletten mit 25 mg (+)-5,11-Dihydro-11-[[(1-methyl-4-piperidinyl)amino]carbonyl]-6H-dibenz[b,e]azepin-6-on

Zusammensetzung:

1 Tablette enthält

| | |
|---|---|
| Wirkstoff | 25,0 mg |
| Milchzucker | 148,0 mg |
| Kartoffelstärke | 65,0 mg |
| Magnesiumstearat | 2,0 mg |
| | 240,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.
Tablettengewicht: 240 mg
Stempel: 9 mm

Beispiel II

Dragées mit 25 mg (+)-5,11-Dihydro-11-[[(1-methyl-4-piperidinyl)amino]carbonyl]-6H-dibenz[b,e]azepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert. Dragéegewicht: 300 mg

Beispiel III

Ampullen mit 1 mg (+)-5,11-Dihydro-11-[[(1-methyl-4-piperidinyl)amino]carbonyl]-6H-dibenz[b,e]azepin-6-on-hydrochlorid

Zusammensetzung:

1 Ampulle enthält:

| Wirkstoff | 1,0 mg |
|---|---|
| Natriumchlorid | 8,0 mg |
| Dest. Wasser ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120° C.

Beispiel IV

Suppositorien mit 25 mg 5,11-Dihydro-11-[(4-methyl-1-piperazinyl)acetyl]-6H-dibenz[b,e]azepin-6-on

Zusammensetzung:

1 Zäpfchen enthält:

| Wirkstoff | 5,0 mg |
|---|---|
| Zäpfchenmasse (z.B. Witepsol W 45 [R]) | 1 695,0 mg |
| | 1 700,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.
Zäpfchengewicht: 1,7 g

Beispiel V

Tropfen mit 0,5 g 5,11-Dihydro-11-[[(1-methyl-4-piperidinyl)amino]carbonyl]-6H-dibenz[b,e]azepin-6-on-hydrochlorid

Zusammensetzung:

100 ml Tropflösung enthalten:

| p-Hydroxybenzoesäuremethylester | | 0,035 g |
|---|---|---|
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

Beispiel VI

Dosieraerosol mit 100 μg 5,11-Dihydro-11-[[(1-methyl-4-piperidinyl)amino]carbonyl]-6H-dibenz[b,e]azepin-6-on-hydrochlorid

1 Dose (150 Hübe) enthält:

| Wirksubstanz | 15,0 mg |
|---|---|
| Äthanol | 990,0 mg |
| Treibgas 12/14 (60:40, v:v) | 8 895,0 mg |
| | 9 900,0 mg |

Herstellungsverfahren:

Der Wirkstoff wird in Äthanol gelöst, die Lösung auf -30°C abgekühlt und in eine vorgekühlte Aluminiumdose eingefüllt. Anschließend wird das auf -50°C abgekühlte Treibgasgemisch zudosiert, das

8

Ventil aufgesetzt und sofort gebördelt.

1 Hub enthält 0,1 mg 5,11-Dihydro-11-[[(1-methyl-4-piperidinyl)amino]carbonyl]-6H-dibenz[b,e]azepin-6-on-hydrochlorid.

**Ansprüche**

1.) Verwendung von 5,11-Dihydro-11-[[(1-methyl-4-piperidinyl)amino]carbonyl]-6H-dibenz[b,e]azepin-6-on und/oder 5,11-Dihydro-11-[(1-methyl-4-piperidinyl)acetyl]-6H-dibenz[b,e]azepin-6-on und/oder 5,11-Dihydro-11-[(4-methyl-1-piperazinyl)acetyl]-6H-dibenz[b,e]azepin-6-on bzw. von physiologisch wirksamen Enantiomeren bzw. von physiologisch verträglichen Salzen dieser Verbindungen mit anorganischen oder organischen Säuren zur Behandlung von akuten und chronischen obstruktiven Atemwegserkrankungen.

2.) Verwendung von 5,11-Dihydro-11-[[(1-methyl-4-piperidinyl)amino]carbonyl]-6H-dibenz[b,e]azepin-6-on und/oder 5,11-Dihydro-11-[(1-methyl-4-piperidinyl)acetyl]-6H-dibenz[b,e]azepin-6-on und/oder 5,11-Dihydro-11-[(4-methyl-1-piperazinyl)acetyl]-6H-dibenz[b,e]azepin-6-on bzw. von physiologisch wirksamen Enantiomeren bzw. von physiologisch verträglichen Salzen dieser Verbindungen mit anorganischen oder organischen Säuren zur Herstellung eines Arzneimittels zur Behandlung von akuten und chronischen obstruktiven Atemwegserkrankungen.

3.) Verwendung der in den Ansprüchen 1 und 2 genannten Verbindungen zur Bekämpfung von Asthma, chronischer Bronchitis und Emphysem.

4.) Verwendung von (+)-5,11-Dihydro-11-[[(1-methyl-4-piperidinyl)amino]-carbonyl]-6H-dibenz[b,e]-azepin-6-on oder dessen physiologisch verträglichen Salzen mit anorganischen oder organischen Säuren zur Behandlung von akuten und chronischen obstruktiven Atemwegserkrankungen.

5.) Verwendung von (+)-5,11-Dihydro-11-[[(1-methyl-4-piperidinyl)amino]-carbonyl]-6H-dibenz[b,e]-azepin-6-on oder dessen physiologisch verträglichen Salzen mit anorganischen oder organischen Säuren zur Herstellung eines Arzneimittels zur Behandlung von akuten und chronischen obstruktiven Atemwegserkrankungen.

6.) Verwendung der Substanz des Anspruchs 4 zur Bekämpfung von Asthma, chronischer Bronchitis und Emphysem.